# EUROPEAN PATENT APPLICATION

(11) **EP 4 032 430 A1**
(43) Date of publication of application: **27.07.2022**
(21) Application number: 20865350.1
(22) Date of filing: 16.09.2020
(51) Int. Cl.: A45D 34/02, C08F 210/02, C08F 214/26, C08F 214/28, F16L 11/06

(54) **FLUORINE-CONTAINING COPOLYMER**

(30) Priority: 20.09.2019 JP 2019171294; 16.10.2019 JP 2019189130; 22.01.2020 JP 2020008109
(71) Applicant: Daikin Industries, Ltd., Osaka-shi, Osaka 530-8323 (JP)
(72) Inventor: FUKAGAWA, Ryouichi, Osaka-shi, Osaka 530-8323 (JP); ISAKA, Tadaharu, Osaka-shi, Osaka 530-8323 (JP); SHIMADA, Hiroyuki, Osaka-shi, Osaka 530-8323 (JP)
(74) Representative: Hoffmann Eitle
(86) International application number: PCT/JP2020/035088
(87) International publication number: WO 2021/054363

(57) **Abstract**

Provided is a fluorine-containing copolymer comprising tetrafluoroethylene units (a), ethylene units (b), and hexafluoropropylene units (c), a content of the hexafluoropropylene units (c) in the total monomer units being 6 to 21% by mole; a mole ratio of the tetrafluoroethylene units (a) to the sum of the tetrafluoroethylene units (a) and the ethylene units (b), (a)/((a) + (b)), being 0.40 to 0.54; and a melt flow rate measured under the conditions of 230°C and a load of 5 kg being 12 to 100 g/10 minutes.

## Description

### TECHNICAL FIELD

The present disclosure relates to a fluorine-containing copolymer.

### BACKGROUND ART

Since fluorine-containing copolymers are excellent in transparency as well as in heat resistance, chemical resistance, etc., they have been used in various applications including optical ones.

For example, Patent Document 1 discloses a fluorine-containing copolymer including 60% by mole or less of copolymerized units of tetrafluoroethylene, 40 to 60% by mole of copolymerized units of ethylene, and 0 to 15% by mole of an α-olefinic monomer copolymerizable with tetrafluoroethylene and ethylene, and a method for producing such a fluorine-containing copolymer.

### RERATED ART

### PATENT DOCUMENTS

Patent Document 1: U.S. Patent No. 4,338,237

### SUMMARY OF THE INVENTION

### PROBLEM TO BE SOLVED BY THE INVENTION

An object of the present disclosure is to provide a fluorine-containing copolymer having a low refractive index and a small Haze value, and which is suppressed in coloring and excellent in molding processability.

### MEANS FOR SOLVING THE PROBLEM

The present disclosure provides a fluorine-containing copolymer comprising tetrafluoroethylene units (a), ethylene units (b), and hexafluoropropylene units (c), a content of the hexafluoropropylene units (c) being 6 to 21% by mole in the total monomer units; a mole ratio of the tetrafluoroethylene units (a) to the sum of the tetrafluoroethylene units (a) and the ethylene units (b), (a)/((a) + (b)), being 0.40 to 0.54; and a melt flow rate measured under the conditions of 230°C and a load of 5 kg being 12 to 100 g/10 minutes.

It is preferable that the fluorine-containing copolymer of the present disclosure comprises 0 to 10% by mole of units of a monomer copolymerizable with tetrafluoroethylene, ethylene, and hexafluoropropylene in the total monomer units.

It is preferable in the fluorine-containing copolymer of the present disclosure that the content of the hexafluoropropylene units (c) in the total monomer units is 9 to 21% by mole.

It is preferable in the fluorine-containing copolymer of the present disclosure that the content of the hexafluoropropylene units (c) in the total monomer units is 10.5 to 18.5% by mole.

It is preferable that the fluorine-containing copolymer of the present disclosure has a melt flow rate of 25 to 50 g/10 minutes measured under the conditions of 230°C and a load of 5 kg.

It is preferable that the fluorine-containing copolymer of the present disclosure has a melting point of 155 to 170°C.

### EFFECTS OF INVENTION

According to the present disclosure, it is possible to provide a fluorine-containing copolymer having a low refractive index and a small Haze value, and which is suppressed in coloring and excellent in molding processability.

### DESCRIPTION OF EMBODIMENTS

Hereinafter, specific embodiments of the present disclosure will be described in more detail. However, the present disclosure is not intended to be limited to the embodiments below.

A fluorine-containing copolymer of the present disclosure is the fluorine-containing copolymer comprising tetrafluoroethylene units (a), ethylene units (b), and hexafluoropropylene units (c),
a content of the hexafluoropropylene units (c) in the total monomer units being 6 to 21% by mole;
a mole ratio of the tetrafluoroethylene units (a) to the sum of the tetrafluoroethylene units (a) and the ethylene units (b), (a)/((a) + (b)), being 0.40 to 0.54; and
a melt flow rate measured under the conditions of 230°C and a load of 5 kg being 12 to 100 g/10 minutes.

The fluorine-containing copolymer of the present disclosure comprises the hexafluoropropylene units (c) at a content of 6 to 21% by mole in the total monomer units, and the content of the hexafluoropropylene units (c) is preferably 9 to 21% by mole, more preferably 10.5 to 18.5% by mole, even more preferably 12 to 17.5% by mole, particularly preferably 13 to 16% by mole. If the content of the hexafluoropropylene units (c) is too low, the Haze value of the fluorine-containing copolymer is large, thereby becoming poor in transparency. On the other hand, if the content of the hexafluoropropylene units (c) is too high, there occurs coloring in the fluorine-containing copolymer.

Further, in the fluorine-containing copolymer of the present disclosure, the mole ratio of the tetrafluoroethylene units (a) to the sum of the tetrafluoroethylene units (a) and the ethylene units (b), (a)/((a)+(b)), i.e., the content in terms of moles of the tetrafluoroethylene units (a)/(the content in terms of moles of the tetrafluoroethylene units (a) + the content in terms of moles of the ethylene units (b)) is 0.40 to 0.54, preferably 0.42 to 0.52, more preferably 0.44 to 0.51, even more preferably 0.46 to 0.50. If the mole ratio, (a)/((a) + (b)), is too low, or too high, there occurs coloring in the fluorine-containing copolymer.

The content of the tetrafluoroethylene units (a) in the total monomer units constituting the fluorine-containing copolymer of the present disclosure is not limited, but preferably 36.0 to 45.0% by mole, more preferably 38.0 to 43.0% by mole, even more preferably 39.0 to 42.0% by mole. By setting the content of the tetrafluoroethylene units (a) within the above range, it is possible to enhance effects of suppressing coloring.

The content of the ethylene units (b) in the total monomer units constituting the fluorine-containing copolymer of the present disclosure is not limited, but preferably 41.0 to 50.0% by mole, more preferably 43.0 to 48.0% by mole, even more preferably 44.0 to 47.0% by mole. By setting the content of the ethylene units (b) within the above range, it is possible to enhance effects of suppressing coloring.

Further, it is preferable that the fluorine-containing copolymer of the present disclosure comprises units of a monomer copolymerizable with tetrafluoroethylene, ethylene, and hexafluoropropylene. A content of units of such a copolymerizable monomer relative to the total polymerized units is preferably 0 to 10% by mole, more preferably 0.05 to 3% by mole, even more preferably 0.3 to 1.0% by mole.

The copolymerizable monomer giving units of such a copolymerizable monomer is not limited, but preferably at least one selected from the group consisting of ethylenically unsaturated monomers represented by the following formulae (1) and (2), but tetrafluoroethylene and hexafluoropropylene are excluded.

Formula (1): CX¹X²=CX³(CF₂)ₙX⁴

wherein X¹, X², X³, and X⁴ are the same or different and represent H, F, or Cl; and n represents an integer of 0 to 8; but tetrafluoroethylene and hexafluoropropylene are excluded.

Formula (2): CF₂=CF-ORf¹

wherein Rf¹ represents an alkyl group having 1 to 3 carbon atoms or a fluoroalkyl group having 1 to 3 carbon atoms.

The ethylenically unsaturated monomer represented by formula (1) is preferably at least one selected from the group consisting of CF₂=CFCl, the following formula (3) :

CH₂=CF-(CF₂)ₙX⁴ (3)

wherein X⁴ represents H, F, or Cl, and n represents an integer of 0 to 8; and the following Formula (4):

CH₂=CH-(CF₂)ₙX⁴ (4)

wherein X⁴ represents H, F, or Cl, and n represents an integer of 0 to 8;
more preferably at least one selected from the group consisting of CF₂=CFCl, CH₂=CFCF₃, CH₂=CH-C₄F₉, CH₂=CH-C₆F₁₃, and CH₂=CF-C₃F₆H; even more preferably at least one selected from the group consisting of CF₂=CFCl, CH₂=CH-C₆F₁₃, CH₂=CFCF₃, and CH₂=CF-C₃F₆H; particularly preferably CH₂=CF-C₃F₆H, i.e., 2,3,3,4,4,5,5-heptafluoro-1-pentene (CH₂=CFCF₂CF₂CF₂H).

The ethylenically unsaturated monomer represented by formula (2) is preferably at least one selected from the group consisting of CF₂=CF-OCF₃, CF₂=CF-OCF₂CF₃, and CF₂=CF-OCF₂CF₂CF₃.

A content of each monomer unit in the fluorine-containing copolymer of the present disclosure can be calculated by appropriately combining, e.g., NMR and elemental analysis depending on a type of a monomer unit constituting the fluorine-containing copolymer.

The fluorine-containing copolymer of the present disclosure has a melt flow rate, MFR, of 12 to 100 g/10 minutes, preferably 25 to 50 g/10 minutes, measured under the conditions of 230°C and a load of 5 kg. When the melt flow rate is too high, the strength is decreased, causing chipping and cracking to occur during molding, thereby becoming insufficient in the strength when the fluorine-containing copolymer is used as various molded bodies etc. On the other hand, when the melt flow rate is too low, molding processability is reduced, thereby becoming difficult to mold to a desired shape.

The melt flow rate, MFR, can be measured by using a melt indexer, in accordance with ASTM D1238. Specifically, it can be obtained as a mass quantity, g/10 minutes, of polymer flowing out of a nozzle having an inner diameter of 2.1 mm and a length of 8 mm for 10 minutes at 230°C under a load of 5 kg.

The fluorine-containing copolymer of the present disclosure has a melting point of preferably 140 to 185°C, more preferably 150 to 175°C, even more preferably 155 to 170°C.

The melting point can be measured in accordance with ASTM D4591 by using a differential scanning calorimeter. Specifically, by using a differential scanning calorimeter, thermal measurement is conducted at a temperature-increasing rate of 10°C/minute, and a temperature corresponding to a peak of an endothermic curve obtained can be determined as the melting point.

The fluorine-containing copolymer of the present disclosure can be produced in the presence of a polymerization initiator by copolymerizing tetrafluoroethylene, ethylene and hexafluoropropylene, and a monomer copolymerizable with these which is used as needed.

The copolymerization may be solution polymerization, bulk polymerization, emulsion polymerization, suspension polymerization, etc., but it is preferably the emulsion polymerization or the suspension polymerization, more preferably the suspension polymerization, in that it is industrially easy to implement.

The polymerization initiator may be an oil-soluble radical polymerization initiator or a water-soluble radical polymerization initiator, but preferable is the oil-soluble radical polymerization initiator.

The oil-soluble radical polymerization initiator may be a known oil-soluble peroxide, and examples typically include:
dialkyl peroxycarbonates such as dinormalpropyl peroxydicarbonate, diisopropyl peroxydicarbonate, and di-sec-butyl peroxydicarbonate;
peroxyesters such as t-butyl peroxyisobutyrate and t-butyl peroxypivalate;
dialkyl peroxides such as di-t-butyl peroxide; and
di[fluoro(or fluorochloro)acyl] peroxides.

Examples of di[fluoro(or fluorochloro)acyl] peroxides include di-acyl peroxides represented by [(RfCOO)-]₂, wherein Rf represents a perfluoroalkyl group, a ω-hydroperfluoroalkyl group, or a fluorochloroalkyl group.

Examples of di[fluoro(or fluorochloro)acyl] peroxides include di(ω-hydro-dodecafluorohexanoyl) peroxide, di(ω-hydro-tetradecafluoroheptanoyl) peroxide, di(ω-hydro-hexadecafluorononanoyl) peroxide, di(perfluorobutyryl) peroxide, di(perfluorovaleryl) peroxide, di(perfluorohexanoyl) peroxide, di(perfluoroheptanoyl) peroxide, di(perfluorooctanoyl) peroxide, di(perfluorononanoyl) peroxide, di(ω-chloro-hexafluorobutyryl) peroxide, di(ω-chloro-decafluorohexanoyl) peroxide, di(ω-chloro-tetradecafluorooctanoyl) peroxide, ω-hydro-dodecafluoroheptanoyl-ω-hydrohexadecafluorononanoyl-peroxide, ω-chloro-hexafluorobutyryl-ω-chloro-decafluorohexanoyl-peroxide, ω-hydrododecafluoroheptanoyl-perfluorobutyryl-peroxide, di(dichloropentafluorobutanoyl) peroxide, di(trichlorooctafluorohexanoyl) peroxide, di(tetrachloroundecafluorooctanoyl) peroxide, di(pentachlorotetradecafluorodecanoyl) peroxide, and di(undecachlorotriacontafluorodocosanoyl) peroxide.

The water-soluble radical polymerization initiator may be a known water-soluble peroxide, and examples include ammonium salts, potassium salts, and sodium salts of persulfuric acid, perboric acid, perchloric acid, perphosphoric acid, percarbonic acid and the like; t-butyl permaleate; and t-butyl hydroperoxide. A reducing agent such as sulfites and salts of sulfurous acid may be used in combination with peroxides, and an amount of the reducing agent used may be 0.1 to 20 times an amount of the peroxides.

In the copolymerization described above, a surfactant, a chain transfer agent, and a solvent can be used. Conventionally known ones can be used for each.

The surfactant may be a known surfactant, e.g., a nonionic surfactant, an anionic surfactant, a cationic surfactant, etc. Above all, preferable is a fluorine-containing anionic surfactant, and more preferable is a linear or branched fluorine-containing anionic surfactant having 4 to 20 carbon atoms which may contain ether bonded oxygen, i.e., an oxygen atom interposed between carbon atoms. An amount of the surfactant added is preferably 50 to 5,000 ppm based on the water used in the polymerization.

Examples of the chain transfer agent include: hydrocarbons such as ethane, isopentane, n-hexane, and cyclohexane; aromatics such as toluene and xylene; ketones such as acetone; acetate esters such as ethyl acetate and butyl acetate; alcohols such as methanol and ethanol; mercaptans such as methyl mercaptan; and halogenated hydrocarbons such as carbon tetrachloride, chloroform, methylene chloride, and methyl chloride. An amount of the chain transfer agent added may be changed depending on magnitude of the chain transfer constant of a compound used, but is used normally in a range of 0.01 to 20% by mass based on the polymerization solvent.

Examples of the solvent include water and a mixed solvent of water and alcohol.

In the suspension polymerization, a fluorine-based solvent in addition to water may be used. Examples of the fluorine-based solvent include:
hydrochlorofluoroalkanes such as CH₃CClF₂, CH₃CCl₂F, CF₃CF₂CCl₂H, and CF₂ClCF₂CFHCl; chlorofluoroalkanes such as CF₂ClCFClCF₂CF₃ and CF₃CFClCFClCF₃; and
perfluoroalkanes such as perfluorocyclobutane, CF₃CF₂CF₂CF₃, CF₃CF₂CF₂CF₂CF₃, and CF₃CF₂CF₂CF₂CF₂CF₃. Above all, the perfluoroalkanes are preferable. An amount of the fluorine-based solvent used is preferably 10 to 100% by mass based on an aqueous medium from aspects of suspensionability and economy.

A polymerization temperature is not limited, and may be 0 to 100°C. A polymerization pressure is determined as appropriate, depending on other polymerization conditions such as a type, an amount and a vapor pressure of the solvent used, and the polymerization temperature, but may be normally 0 to 9.8 MPaG.

The fluorine-containing copolymer of the present disclosure may be in any form, and may be in aqueous dispersion, powder, pellets, etc.

The fluorine-containing copolymer of the present disclosure has a low refractive index and a small Haze value, and is excellent in transparency, and further suppressed in coloring.

The refractive index of the fluorine-containing copolymer of the present disclosure is preferably 1.340 to 1.375, more preferably 1.345 to 1.370, even more preferably 1.347 to 1.360. The refractive index of the fluorine-containing copolymer can be measured at 25°C with a sodium D line as a light source by using Abbe's refractometer.

Further, the Haze value of the fluorine-containing copolymer of the present disclosure is preferably 50 or less, more preferably 35 or less, even more preferably 20 or less, and the lower limit is preferably 10 or more. The Haze value of the fluorine-containing copolymer can be measured by using a Haze meter with respect to a sheet of the fluorine-containing copolymer having a thickness of 2.0 mm, in accordance with ASTM D1003.

Furthermore, the fluorine-containing copolymer of the present disclosure has a yellow index value (YI value) of preferably 0 or less, more preferably -2 or less, even more preferably -5 or less. The yellow index value can be measured by using a color difference meter in accordance with ASTM D1925.

The fluorine-containing copolymer of the present disclosure can be molded to a variety of molded bodies. The molded body obtained has a low refractive index and a small Haze value, and is suppressed in coloring. Also, since the fluorine-containing copolymer of the present disclosure is excellent in molding processability, when molded to a variety of the molded bodies, molding can be performed very well, to suitably obtain the molded body having a desired shape.

The shape of the above molded body is not limited, and may be e.g., a hose, a pipe, a tube, a sheet, a seal, a gasket, a packing, a film, a tank, a roller, a bottle, and a container.

A method for molding the fluorine-containing copolymer is not limited, and examples include compression molding, extrusion molding, transfer molding, injection molding, rotational molding, rotational lining, and electrostatic coating. In the case that the fluorine-containing copolymer of the present disclosure is molded to a pipe or a tube, the extrusion molding is preferable.

The fluorine-containing copolymer of the present disclosure may be molded after mixing with a filler material, a plasticizing agent, a processing aid, a mold release agent, a pigment, a flame retarder, a lubricant, a light stabilizer, a weather resistant stabilizer, a conductive material, an antistatic agent, an ultraviolet absorbing agent, an antioxidant, a foaming agent, an aroma chemical, oil, a softening agent, and a de-hydrogen fluoride agent. Examples of the filler materials include polytetrafluoroethylene, mica, silica, talc, celite, clay, titanium oxide, and barium sulfate. Examples of the conductive materials include carbon black. Examples of the plasticizing agents include dioctyl phthalate and pentaerythritol. Examples of the processing aids include carnauba wax, a sulfone compound, low molecular weight polyethylene, and a fluorine-based aid. Examples of the de-hydrogen fluoride agents include organic onium and amidines.

Since the fluorine-containing copolymer of the present disclosure has a low refractive index and a small Haze value, and is excellent in transparency and further suppressed in coloring, it can be suitably used as a transparent tube. The transparent tube is not limited, but may be a transparent tube for transferring various liquids. To give an example, in a liquid product that is required to have excellent aesthetic nature of appearance, e.g., in a fragrance product including a liquid fragrance, the fluorine-containing copolymer of the present disclosure can be suitably used as a transparent tube etc. for transferring the liquid fragrance.

Further, the fluorine-containing copolymer of the present disclosure can be suitably utilized as a molding material for molded bodies below.

Namely, examples of these molded bodies include:
food packaging films, lining materials of fluid transfer lines used in food production processes, and fluid transfer members for a food production apparatus such as packings, sealing materials, and sheets;
plugs and packaging films for chemicals, lining materials of fluid transfer lines used in chemicals production processes, and chemical solution transfer members such as packings, sealing materials, and sheets;
inner surface lining members of a chemical solution tank and a piping in a chemical plant and a semiconductor plant;
O (square)-rings/tubes/packings, valve core materials, hoses, sealing materials, etc. used for fuel systems and peripheral equipment of automobiles, and fuel transfer members of hoses, sealing materials, etc. used for AT devices of the automobiles;
a flange gasket of a carburetor, a shaft seal, a valve stem seal, a sealing material, a hose, etc. used for engines and peripheral equipment of automobiles, brake hoses, air conditioner hoses, and radiator hoses of the automobiles, and other automobile part members of electric wire covering materials, etc.;
O (square)-rings, tubes, and packings of the semiconductor production equipment, and chemical solution transfer members for the semiconductor production equipment such as valve core materials, hoses, sealing materials, rolls, gaskets, diaphragms, and pipe fittings;
painting rolls, hoses, and tubes for painting equipment, and painting/ink members of ink containers, etc.;
food and drink transfer members such as tubes and hoses such as tubes for food and drink or hoses for food and drink, belts, packings, and pipe fittings, food packaging materials, and glass cooking equipment;
waste liquid transportation members such as tubes and hoses for waste liquid transportation;
high temperature liquid transportation members such as tubes and hoses for high temperature liquid transportation;
steam piping members such as tubes and hoses for steam piping;
anti-corrosion tapes for piping such as tapes wrapped around piping on ship decks etc.;
various covering materials such as electric wire covering materials, optical fiber covering materials, transparent front-side covering materials provided on a front side of a light incidence side of a photovoltaic element of a solar cell, and back side agents
sliding members such as diaphragms of a diaphragm pump, and various packings;
agricultural films, and weather resistance covers such as various roof materials/side wall materials;
interior materials used in an architectural field, and covering materials for glasses such as a non-flammable fireproof safety glass;
lining materials for a laminated steel plate etc. used in a field of a home appliance, etc.;
riser pipes transporting resources from the sea bottom to the sea surface in an offshore oil field or gas field; and
coating materials and lining materials of innermost surfaces and outermost surfaces of fluid transporting metal piping for crude oils and natural gases.

Examples of the fuel transfer members used for the fuel systems of the automobiles described above further include a fuel hose, a filler hose, and an evaporator hose. The fuel transfer members can be used for fuel containing gasoline additives such as sour gasoline resistant additives, alcohol fuel resistant additives, and methyl tertiary-butyl ether resistant/amine resistant additives.

The plugs/packaging films for the chemicals described above have excellent chemical resistance against acid and the like. Further, examples of the chemical solution transfer members include anti-corrosion tapes wrapped around piping of chemical plant.

Examples of the molded bodies described above include a radiator tank for automobiles, a chemical solution tank, bellows, a spacer, a roller, a gasoline tank, a container for the waste liquid transportation, a container for the high temperature liquid transportation, and tanks for fishery/fish farming.

Examples of the molded bodies further include members used for an automobile bumper, a door trim, a gauge board, a food processing apparatus, a cooking appliance, a water- and oil-repellency glass, a lightening related equipment, a display panel/housing of OA equipment, an illuminated signboard, a display, a liquid crystal display, a mobile phone, a printed circuit board, an electric/electronic component, a general merchandise, a trash can, a bathtub, a bath module, a ventilating fan, and a frame for lightening.

Further, the fluorine-containing copolymer of the present disclosure can be a powder coating material composed of the fluorine-containing copolymer. Such a powder coating material may have an average particle size of 10 to 500 µm. The average particle size can be measured by using a laser diffraction particle size distribution analyzer. After the powder coating material is sprayed on a substrate by an electrostatic coating, a coating film without a foaming trace can be obtained by sintering.

Although the embodiments have been described above, it will be appreciated that various modifications of the embodiments and details are possible without departing from the effect and scope of the claims.

### EXAMPLES

Hereinafter, the embodiments of the present disclosure will be described with reference to Examples. However, the present disclosure is not intended to be limited only to the Examples.

Values in the Examples was measured by the following method.

### <Monomer Composition>

Using powder of the fluorine-containing copolymer and a nuclear magnetic resonance instrument AC300 manufactured by Bruker-Biospin, ¹⁹F-NMR measurement was conducted at a measurement temperature of (a melting point of a polymer + 20)°C, to obtain a monomer composition from an integrated value of each peak. Also, on this occasion, depending on a type of the monomer, an elemental analysis was combined as appropriate.

### <Melt Flow Rate (MFR)>

In accordance with ASTM D1238, using pellets of the fluorine-containing copolymer and the melt indexer manufactured by Yasuda Seiki Seisakusho, Ltd., a mass quantity, g/10 minutes, of the copolymer flowing out of a nozzle having an inner diameter of 2.1 mm and a length of 8 mm for 10 minutes at 230°C under a load of 5 kg was obtained.

### <Melting Point>

With respect to the powder of the fluorine-containing copolymer, using a differential scanning calorimeter RDC220 manufactured by Seiko Instruments Inc., in accordance with ASTM D4591, a thermal measurement was conducted at a temperature-increasing rate of 10°C/minute and the melting point was obtained from a peak of an endothermic curve obtained.

### <Refractive Index>

Pellets of the fluorine-containing copolymer were compressively molded to a film having a thickness of 200 µm. With respect to the sheet-like molded body obtained, the refractive index was measured at 25°C with a sodium D line as a light source by using Abbe's refractometer manufactured by Atago Optical Instrument Co., Ltd.

### <Haze Value>

The pellets of the fluorine-containing copolymer were compressively molded to a film having a thickness of 2.0 mm. With respect to the sheet-like molded body obtained, the Haze value was measured by using a Haze meter, Haze Gard II, manufactured by Toyo Seiki Seisakusho, Ltd. in accordance with ASTM D1003.

### <Yellow Index Value (YI Value)>

The pellets of the fluorine-containing copolymer were put in a case for reflection measurement, and a yellow index value (YI value) was measured by using a color difference meter, ZE6000, manufactured by Nippon Denshoku Industries Co., Ltd., in accordance with ASTM D1925.

### Example 1

After adding 52 L of distilled water to an autoclave and sufficiently conducting nitrogen replacement, 13 kg of perfluorocyclobutane, 26 kg of hexafluoropropylene, and 0.1 kg of 2,3,3,4,4,5,5-heptafluoro-1-pentene, i.e., CH₂=CFCF₂CF₂CF₂H, were placed in the autoclave. The system was heated to 35°C and stirred, and held at 35°C and a stirring speed of 200 rpm. Next, 5.4 kg of tetrafluoroethylene and further 0.2 kg of ethylene were pressed in, and then 0.5 kg of di-n-propyl peroxycarbonate was added, to start polymerization. Since an internal pressure of the system was decreased as the polymerization progressed, a mixed gas of tetrafluoroethylene/ethylene/hexafluoropropylene = 41/44.5/14.5% by mole was continuously supplied and the stirring was continued for 30 hours while holding the system pressure at 1.15 MPa. Next, the pressure was released and returned to atmospheric pressure, and then the reaction product was washed with water and dried to obtain 40 kg of powder of a fluorine-containing copolymer. The monomer composition of the fluorine-containing copolymer obtained is shown in Table 1. Then the obtained powder of the fluorine-containing copolymer was extruded at a cylinder temperature of 255°C by using a single screw extruder, VS50-24, manufactured by Tanabe Plastics Machinery Co., Ltd. to obtain pellets of the fluorine-containing copolymer. According to the above methods, each evaluation was conducted. The evaluation results are shown in Table 1.

### Example 2

Fluorine-containing copolymer powder and pellets were obtained in the same way as in Example 1 and evaluated similarly, except that the amounts of the perfluorocyclobutane and the hexafluoropropylene added at the start of the polymerization were respectively changed to 25 kg and 14 kg, and that the ratio of the mixed gas supplied during progressing of the polymerization reaction was changed to tetrafluoroethylene/ethylene/hexafluoropropylene = 44/48/8% by mole. The monomer composition of the obtained fluorine-containing copolymer and the evaluation results are shown in Table 1.

### Example 3

Fluorine-containing copolymer powder and pellets were obtained in the same way as in Example 1 and evaluated similarly, except that the amounts of the perfluorocyclobutane and the hexafluoropropylene added at the start of the polymerization were respectively changed to 22 kg and 17 kg, and that the ratio of the mixed gas supplied during progressing of the polymerization reaction was changed to tetrafluoroethylene/ethylene/hexafluoropropylene = 43/47.5/9.5% by mole. The monomer composition of the obtained fluorine-containing copolymer and the evaluation results are shown in Table 1.

### Example 4

Fluorine-containing copolymer powder and pellets were obtained in the same way as in Example 1 and evaluated similarly, except that the amounts of the perfluorocyclobutane and the hexafluoropropylene added at the start of the polymerization were respectively changed to 17 kg and 22 kg, and that the ratio of the mixed gas supplied during progressing of the polymerization reaction was changed to tetrafluoroethylene/ethylene/hexafluoropropylene = 42/46/12% by mole. The monomer composition of the obtained fluorine-containing copolymer and the evaluation results are shown in Table 1.

### Example 5

Fluorine-containing copolymer powder and pellets were obtained in the same way as in Example 1 and evaluated similarly, except that the amounts of the perfluorocyclobutane and the hexafluoropropylene added at the start of the polymerization were respectively changed to 7 kg and 32 kg, and that the ratio of the mixed gas supplied during progressing of the polymerization reaction was changed to tetrafluoroethylene/ethylene/hexafluoropropylene = 39/43/18% by mole. The monomer composition of the obtained fluorine-containing copolymer and the evaluation results are shown in Table 1.

### Example 6

Fluorine-containing copolymer powder and pellets were obtained in the same way as in Example 1 and evaluated similarly, except that the ratio of the mixed gas supplied during progressing of the polymerization reaction was changed to tetrafluoroethylene/ethylene/hexafluoropropylene = 37/48/15% by mole. The monomer composition of the obtained fluorine-containing copolymer and the evaluation results are shown in Table 1.

### Example 7

Fluorine-containing copolymer powder and pellets were obtained in the same way as in Example 1 and evaluated similarly, except that the ratio of the mixed gas supplied during progressing of the polymerization reaction was changed to tetrafluoroethylene/ethylene/hexafluoropropylene = 43/42/15% by mole. The monomer composition of the obtained fluorine-containing copolymer and the evaluation results are shown in Table 1.

### Comparative Example 1

Fluorine-containing copolymer powder and pellets were obtained in the same way as in Example 1 and evaluated similarly, except that the perfluorocyclobutane was not used and the amount of the hexafluoropropylene added at the start of the polymerization was changed to 39 kg, and that the ratio of the mixed gas supplied during progressing of the polymerization reaction was changed to tetrafluoroethylene/ethylene/hexafluoropropylene = 37/41/22% by mole. The monomer composition of the obtained fluorine-containing copolymer and the evaluation results are shown in Table 1.

### Comparative Example 2

Fluorine-containing copolymer powder and pellets were obtained in the same way as in Example 1 and evaluated similarly, except that the amount of the perfluorocyclobutane added at the start of the polymerization was changed to 39 kg, and the hexafluoropropylene and the 2,3,3,4,4,5,5-heptafluoro-1-pentene were not used, and that the ratio of the mixed gas supplied during progressing of the polymerization reaction was changed to tetrafluoroethylene/ethylene = 48/52% by mole. The monomer composition of the obtained fluorine-containing copolymer and the evaluation results are shown in Table 1.

### Comparative Example 3

Fluorine-containing copolymer powder and pellets were obtained in the same way as in Example 1 and evaluated similarly, except that the ratio of the mixed gas supplied during progressing of the polymerization reaction was changed to tetrafluoroethylene/ethylene/hexafluoropropylene = 32/53/15% by mole. The monomer composition of the obtained fluorine-containing copolymer and the evaluation results are shown in Table 1.

### Comparative Example 4

Fluorine-containing copolymer powder and pellets were obtained in the same way as in Example 1 and evaluated similarly, except that the ratio of the mixed gas supplied during progressing of the polymerization reaction was changed to tetrafluoroethylene/ethylene/hexafluoropropylene = 48/37.5/14.5% by mole. The monomer composition of the obtained fluorine-containing copolymer and the evaluation results are shown in Table 1.

### [Table 1]

**Table 1**

| | | | Example 1 | Example 2 | Example 3 | Example 4 | Example 5 | Example 6 | Example 7 | Comparative Example 1 | Comparative Example 2 | Comparative Example 3 | Comparative Example 4 |
|---|---|---|---|---|---|---|---|---|---|---|---|---|---|
| Monomer Composition | TFE Unit | (% by mole) | 40.5 | 43.6 | 42.9 | 41.7 | 38.8 | 37.4 | 43.4 | 37.2 | 47.6 | 32.3 | 47.6 |
| | Et Unit | (% by mole) | 44.5 | 47.9 | 47.1 | 45.8 | 42.7 | 47.6 | 41.6 | 40.8 | 52.4 | 52.7 | 37.4 |
| | HFP Unit | (% by mole) | 14.5 | 8.0 | 9.5 | 12.0 | 18.0 | 14.5 | 14.5 | 21.5 | 0.0 | 14.5 | 14.5 |
| | H2P Unit | (% by mole) | 0.5 | 0.5 | 0.5 | 0.5 | 0.5 | 0.5 | 0.5 | 0.5 | 0.0 | 0.5 | 0.5 |
| | TFE Unit/(TFE Unit + Et Unit) | | 0.48 | 0.48 | 0.48 | 0.48 | 0.48 | 0.44 | 0.51 | 0.48 | 0.48 | 0.38 | 0.56 |
| Melt Flow Rate | | (g/10 minutes) | 37 | 26 | 40 | 30 | 49 | 44 | 28 | 49 | 23 | 26 | 44 |
| Melting Point | | (°C) | 162 | 212 | 200 | 181 | 135 | 168 | 156 | 108 | 274 | 178 | 148 |
| Refractive Index | | | 1.353 | 1.367 | 1.364 | 1.358 | 1.347 | 1.355 | 1.352 | 1.341 | 1.388 | 1.359 | 1.349 |
| Haze Value | | | 14.7 | 43.6 | 38.9 | 25.5 | 12.5 | 15.5 | 13.3 | 9.5 | 95 | 20.2 | 12.5 |
| Yellow Index Value (YI Value) | | | -6 | -10 | -3 | -7 | -2 | -5 | -4 | 1 | -11 | 10 | 5 |

In Table 1, the TFE unit indicates the tetrafluoroethylene unit, the Et unit indicates the ethylene unit, the HFP unit indicates the hexafluoropropylene unit, and the H2P unit indicates the 2,3,3,4,4,5,5-heptafluoro-1-pentene unit.

## Claims

1. A fluorine-containing copolymer comprising tetrafluoroethylene units (a), ethylene units (b), and hexafluoropropylene units (c),
a content of the hexafluoropropylene units (c) in the total monomer units being 6 to 21% by mole;
a mole ratio of the tetrafluoroethylene units (a) to the sum of the tetrafluoroethylene units (a) and the ethylene units (b), (a)/((a) + (b)), being 0.40 to 0.54; and
a melt flow rate measured under the conditions of 230°C and a load of 5 kg being 12 to 100 g/10 minutes.

2. The fluorine-containing copolymer according to claim 1, wherein the fluorine-containing copolymer comprises 0 to 10% by mole of units of a monomer copolymerizable with tetrafluoroethylene, ethylene, and hexafluoropropylene in the total monomer units.

3. The fluorine-containing copolymer according to claim 1 or 2, wherein the content of the hexafluoropropylene units (c) in the total monomer units is 9 to 21% by mole.

4. The fluorine-containing copolymer according to any one of claims 1 to 3, wherein the content of the hexafluoropropylene units (c) in the total monomer units is 10.5 to 18.5% by mole.

5. The fluorine-containing copolymer according to any one of claims 1 to 4, wherein the melt flow rate measured under the conditions of 230°C and a load of 5 kg is 25 to 50 g/10 minutes.

6. The fluorine-containing copolymer according to any one of claims 1 to 5, wherein a melting point is 155 to 170°C.
